# EUROPEAN PATENT SPECIFICATION

(11) **EP 4 035 740 B1**
(45) Date of publication and mention of the grant of the patent: **22.03.2023**
(21) Application number: 21211851.7
(22) Date of filing: 02.12.2021
(51) Int. Cl.: A62B 18/02, A62B 27/00, A62B 18/08, B01D 15/02, B01D 24/28, B01D 24/34, B01D 33/00, B01D 46/32

(54) **SYSTEM AND METHOD FOR CONTEXT AWARE NETWORKED MASK WITH DYNAMIC AIR IMPEDANCE FOR OPTIMUM BREATHABILITY**
SYSTEM UND VERFAHREN FÜR KONTEXTBEWUSSTE VERNETZTE MASKE MIT DYNAMISCHER LUFTIMPEDANZ FÜR OPTIMALE ATMUNGSAKTIVITÄT
SYSTÈME ET PROCÉDÉ POUR MASQUE EN RÉSEAU SENSIBLE AU CONTEXTE À IMPÉDANCE D'AIR DYNAMIQUE POUR RESPIRABILITÉ OPTIMALE

(30) Priority: 28.01.2021 IN 202121003902
(43) Date of publication of application: 03.08.2022
(73) Proprietor: Tata Consultancy Services Limited, Maharashtra (IN)
(72) Inventor: KIMBAHUNE, SANJAY MADHUKAR, 400601 Thane (West), Maharashtra (IN); GHOSE, AVIK, 700160 Kolkata, West Bengal (IN); SHINDE, SUJIT RAGHUNATH, 400601 Thane, Maharashtra (IN); INDANI, ASHISH, 400076 Mumbai, Maharashtra (IN); GOULIKAR, DEVRAJ, 400076 Mumbai, Maharashtra (IN); DEY, SWARNAVA, 700160 Kolkata, West Bengal (IN)
(74) Representative: Goddar, Heinz J.

(56) References cited:
- WO-A1-2009/142389
- CN-B- 109 222 281
- KR-A- 20110 014 076
- KR-U- 20110 007 058
- US-A1- 2007 193 584

## Description

### CROSS-REFERENCE TO RELATED APPLICATIONS AND PRIORITY

The present application claims priority from Indian application no. 202121003902, filed on January 28, 2021.

### TECHNICAL FIELD

The embodiments herein generally relate to smart masks and, more particularly, to a system and method for context aware networked mask with dynamic air impedance for optimum breathability.

### BACKGROUND

It has been predicted that masks will be part of everyday life for an extended period, post Covid-19 pandemic era. Masks offer some level of protection against virus. Masks filter out pathogenic particles. As viruses are very small, it is imperative to have reasonably small pore size along with other factors like static charges. It has been observed that prolonged use of mask leads to many issues. For people with breathing difficulties, it is a bigger challenge. Masks like N95 are worn because of fear, however their breathability is also troublesome for common people who are not accustomed to wearing N95 masks. Masks, if worn while exercising, can cause havoc to health. Some masks offer breather unit, but their efficacy is questioned by healthcare community especially for transmitting the virus to others. Thus, it is more comfortable for a user to wear a right mask for a right scenario. However, maintaining multiple masks and rightly identifying the appropriate type of mask to be worn for a current environment is practically challenging for a general user. Moreover, selection of appropriate mask is very much dependent on the health state of the individual and a generalized approach may not be convenient or comfortable to each individual.

Research carried out on smart masks introduce smartness in different contexts and perspectives. One of the existing methods focusses on smartly alerting user by identifying possibility of infectious crowd around, while some other smart masks intelligently guide user through a least polluted route by monitoring the air quality in the ambient environment. Some existing works disclose capability to alert user based on degrading quality of the filter, while other works discuss on intelligent masks that auto trigger cleaning of residual particles settled on the filter area of the mask, with appropriate alerts and suggestions to the user. However, hardly any attempts have been made to introduce smartness in context of breathability of the mask in terms of air impedance variations.

Document KR20110007058U discloses respiratory masks constructed with folds in the filter material, thereby allowing for filter porosity changes, by manually stretching the fabric. This document fails to disclose sensors and automatic stretch control means.

### SUMMARY

Embodiments of the present disclosure present technological improvements as solutions to one or more of the above-mentioned technical problems recognized by the inventors in conventional systems.

For example, a system for context aware networked mask with dynamic air impedance for optimum breathability is provided. The system comprises a mask comprising a base material attached with holding strings at two ends, wherein the base material holds: a mesh network having one or more layers with a pore diameter associated with each of the one or more layers, wherein each of the one or more layers are held by an actuator applying a stretch force on each of the one or more layers; a plurality of sensors , attached to the base material, and comprising a plurality of air quality sensors for sensing a plurality of environmental parameters and a plurality of biological sensors for sensing a plurality of biological parameters of a wearer of the mask; a communication interface attached to the base material and configured to a) transmit the sensed plurality of environmental parameters and the biological parameters to a master device and b) receive a customized risk score from the master device; and an actuator unit configured to process the customized risk score received from the communication module to generate an actuation signal that varies the stretch force applied by the actuator on each of the one or more layers in accordance to the customized risk score to vary resultant air impedance of the mesh network by varying effective pore diameter to adjust breathability for the wearer; the master device comprising: a memory storing instructions; one or more Input/Output (I/O) interfaces; and one or more hardware processors coupled to the memory via the one or more I/O interfaces, wherein the one or more hardware processors are configured by the instructions to: process, via an AI based Real time risk calculation module in the memory implemented by the one or more hardware processors , a) the plurality of environmental parameters, the plurality of biological parameters, ambient environment and context data of the wearer, meta data of the wearer and user inputs to generate the customized risk score; and b) generate alerts for the wearer in accordance to the customized risk score and a set of predefined rules.

The master device is configured to communicate with a cloud server 106 for computationally intensive processing to derive health insights from historical data comprising customized risk scores, the meta data of the wearer, the plurality of biological parameters, the plurality of environmental parameters.

In another aspect, in one embodiment, a method for context aware networked mask with dynamic air impedance for optimum breathability is provided. The method includes receiving, by one or more hardware processors of a master device, a plurality of environmental parameters and a plurality of biological parameters sensed by a plurality of sensors of a mask. Further the method comprises processing, via an AI based Real time risk calculation module implemented by the one or more hardware processors, the plurality of environmental parameters, the plurality of biological parameters, ambient environment and context data of a wearer of the mask, meta data of the wearer and user inputs to generate a customized risk score. Furthermore the method comprises communicating, via the one or more hardware processors, the customized risk score to an actuator unit of the mask, wherein actuator unit processes the customized risk score to generate an actuation signal that varies a stretch force applied by an actuator on each of one or more layers of a mesh network of the mask in accordance with the customized risk score to vary effective pore diameter of the mesh network, which in turn varies resultant air impedance to adjust breathability for the wearer. Furthermore, the method comprises generating, via the one or more hardware processors, alerts for the wearer in accordance with the customized risk score and a set of predefined rules.

The method further comprising communicating with a cloud server for computationally intensive processing to derive health insights from historical data comprising customized risk scores, the meta data of the wearer, the biological plurality of parameters, the plurality of environmental parameters.

In yet another aspect, there are provided one or more non-transitory machine-readable information storage mediums comprising one or more instructions, which when executed by one or more hardware processors causes a method for context aware networked mask with dynamic air impedance for optimum breathability is provided. The method comprises receiving of a master device, a plurality of environmental parameters and a plurality of biological parameters sensed by a plurality of sensors of a mask. Further the method comprises processing, via an AI based Real time risk calculation module the plurality of environmental parameters, the plurality of biological parameters, ambient environment and context data of a wearer of the mask, meta data of the wearer and user inputs to generate a customized risk score. Furthermore the method comprises communicating the customized risk score to an actuator unit of the mask, wherein actuator unit processes the customized risk score to generate an actuation signal that varies a stretch force applied by an actuator on each of one or more layers of a mesh network of the mask in accordance with the customized risk score to vary effective pore diameter of the mesh network, which in turn varies resultant air impedance to adjust breathability for the wearer. Furthermore, the method comprises generating alerts for the wearer in accordance with the customized risk score and a set of predefined rules. The method further comprising communicating with a cloud server for computationally intensive processing to derive health insights from historical data comprising customized risk scores, the meta data of the wearer, the biological parameters, the plurality of environmental parameters.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 is an overview of a system for context aware networked mask with dynamic air impedance for optimum breathability, in accordance with some embodiments of the present disclosure.
FIG. 2 is a functional block diagram of a master device of the system of FIG. 1 for context aware networked mask with dynamic air impedance for optimum breathability, in accordance with some embodiments of the present disclosure.
FIG. 3A through 3C (collectively referred as FIG. 3) is an illustrative example of a single layered mesh network used in the mask of the system of FIG. 1, in accordance with some embodiments of the present disclosure.
FIG. 4A through 4E (collectively referred as FIG. 4) is an illustrative example of a multi-layered mesh network used in the mask of the system of FIG. 1, in accordance with some embodiments of the present disclosure.
FIG. 5 is a flow diagram illustrating a method for context aware networked mask with dynamic air impedance for optimum breathability, using the system of FIG. 1, in accordance with some embodiments of the present disclosure.

It should be appreciated by those skilled in the art that any block diagrams herein represent conceptual views of illustrative systems and devices embodying the principles of the present subject matter. Similarly, it will be appreciated that any flow charts, flow diagrams, and the like represent various processes which may be substantially represented in computer readable medium and so executed by a computer or processor, whether or not such computer or processor is explicitly shown.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

While facing polluted environments one does not need extreme breath filtering protection all the time. It is dependent on many factors such as immunity of the person, type of pollutants around, current physical state of the person and the like. Methods of the art hardly focus on breathability aspect while working on smart masks. Embodiments of the present disclosure provide a system and method for context aware networked mask with dynamic air impedance for optimum breathability. The system provides a smart mask that regulates the air inlet (by modulating the position / pore size of filtering membrane or by using resizable gel) as per the prevalent risk at that instance. The context aware mask with a mesh network, disclosed herein, comprises sensors and processing unit, which use contextual information and Artificial Intelligence to develop models for generating alerts along with mechanism of dynamically regulating the pore size to ensure breathability by adjusting air impedance.

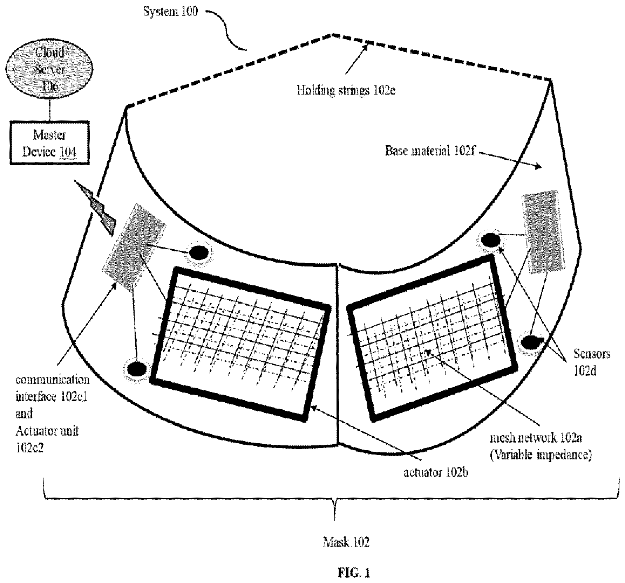

Referring now to the drawings, and more particularly to FIGS. 1 through 5, where similar reference characters denote corresponding features consistently throughout the figures, there are shown preferred embodiments and these embodiments are described in the context of the following exemplary system and/or method.

FIG. 1 is an overview of a system 100 for context aware networked mask with dynamic air impedance for optimum breathability, in accordance with some embodiments of the present disclosure. The system 100 includes a mask 102 worn by a wearer, a master device 104 owned by the wearer and in proximity of the mask and a cloud server 106 connected to the master device 104 through long range communication networks such as cellular or the like. The system 100 provides a smart network enabled mask that is aware of wearers' biological parameters and environment around him. The system 100 dynamically changes the air impedance of the mask 102 based on the context such as risk posed current ambient environment of the wearer in accordance individuals current health state, meta data of the wearer gathered over time and so on . Sensors 102d placed on the mask 102 sense the environment from within the mask as well as outside the mask. A communication interface 102c1 facilitates smooth data exchange across the sensors 102d and the master device 104. Based on user's (wearer's) meta data, instantaneous sensor readings, past historical data and Artificial Intelligence (AI) based processing in the master device 104, appropriate alerts are issued to the wearer. The master device 104 is installed with a mobile app for data entry, visualization and alerting. The master device 104 can be a smart phone, a personal digital assistant or any other device capable of processing the data using AI based modules and communicating with the cloud server 106.

In safety critical deployments e.g. workers in hazardous environment, immune-compromised patients, the master device 104 may need to process data from different orthogonal sensors, with multiple time series streams and generate inference. One artificial intelligence model cannot be trained for all such sensors. Thus, a set of AI models is required. These require resources and computation power even greater than that can be supported by the master device 104, such as the smart phone. Given that the lower level reorientations for time series signature classification is fairly generic, multi objective neural networks can be optimized fixing set of lower layers and a set of high-level layers that can be transfer learned / fine-tuned for each sensor. The determination of this partition is not trivial and is based on the desired latency, processing, memory and also the accuracy especially false negatives that may have telling effect on user health. This optimization is formulated and implemented it in the AI based risk calculation module 210 The AI based risk calculation module 210 can be generated in cloud server and then transferred on the master device 104. The data from the external sensors could be utilized to build super granular air quality/pathogen presence maps in a given area. Such masks would not only enable user to safely navigate through contaminated / risk prone areas, but allow better breathing through mask, when the user is in a relatively safer area.

Referring now to the system of FIG. 1, the mask 102 comprises a base material 102f attached with holding strings 102e at two ends. The base material can be chosen to be any skin friendly flexible material such as cloth or polymer. The base material 102f holds: a mesh network (102a) having one or more layers with a predefined pore size associated with each of the one or more layers, wherein each of the one or more layers is held by an actuator 102b by applying a stretch force on each of the one or more layers. The mesh network can be:
a) A mesh of closely knit polymer with elastic properties, wherein average or effective pore diameter of the closely knit polymer increases with the increasing stretch force applied by the actuator unit allowing more air to enter the mask for increased breathability.
b) A multi-layered mesh with pore diameters (interchangeably referred as pore sizes) larger than the closely knit polymer, wherein layers of the mesh are overlaid on each other, and wherein the actuator unit is configured to realign the pores of each layer in accordance with the actuator control signal to vary the resultant pore size of the multi-layered mesh changing the air impedance and modulating the breathability.

A plurality of sensors 102d are attached to the base material 102f. The sensors 102d are broadly of two types, a plurality of air quality sensors for sensing a plurality of environmental parameters and a plurality of biological sensors for sensing plurality of biological parameters of the wearer. The air quality sensors comprise pathogen sensors, pollen grain sensors, Volatile Organic Compounds (VOC) sensors, bacteria sensors, ear lobe based PPG sensors and mold sensors. The biological sensors comprise CO2 sensors, temperature sensors, SPO2 and moisture sensors.

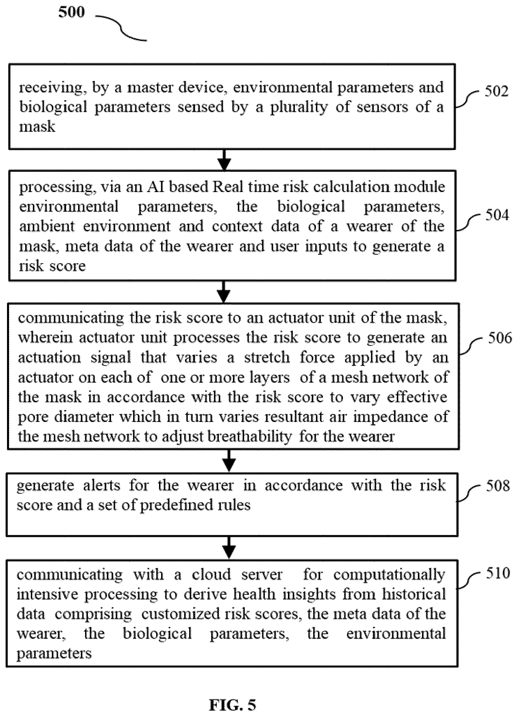

The communication interface (102c1) attached to the base material (102f) is configured to a) transmit the sensed environmental parameters and the biological parameters to a master device and b) receive a customized risk score from the master device. The risk score computation is performed on the master device 104 and is explained later in conjunction with FIG. 5.

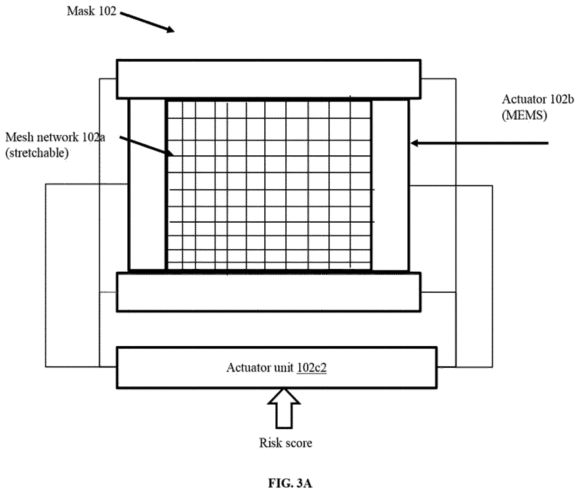

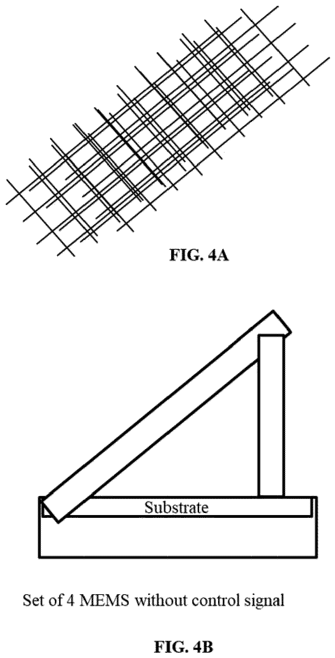

A small actuator unit (102c2) is mounted on the mask and is configured to process the customized risk score received from the communication module (102c1) to generate an actuation signal. The actuation signal varies say a voltage signal that further varies the stretch force applied by the actuator on each of the one or more layers in accordance with the customized risk score. According to the invention, this stretch force varies the pore diameter of the one or more layers, effectively varying resultant air impedance of the mesh network to adjust breathability for the wearer. The actuator is a precision actuator such as Micro ElectroMechanical System (MEMS), piezoelectric crystals or bimetallic strips. An example single layer mesh network with actuator assembly and construction is shown in FIGS. 3A through 3C, and FIGS. 4A through 4E depict a multi-layered mech network with the actuator assembly.

Further, the mask 102 can communicate with the master device 104 configured to process, via an AI based Real time risk calculation module a) the plurality of environmental parameters, the plurality of biological parameters, ambient environment and context data of the wearer, meta data of the wearer and user inputs to generate the customized risk score; and b) generate alerts for the wearer in accordance to the customized risk score and a set of predefined rules. Further, the master device 104 is configured to communicate with a cloud server 106 for computationally intensive processing to derive health insights from historical data comprising customized risk scores, the meta data of the wearer, the plurality of biological parameters, the plurality of environmental parameters.

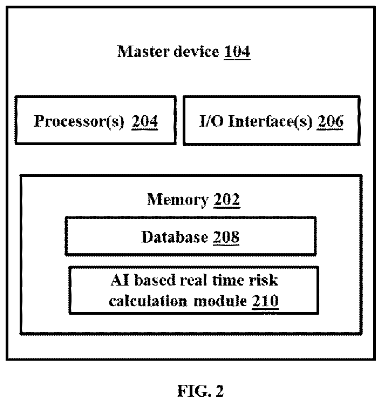

FIG. 2 is a functional block diagram of a master device of the system of FIG. 1 for context aware networked mask with dynamic air impedance for optimum breathability, in accordance with some embodiments of the present disclosure.

In an embodiment, the master device 102 includes a processor(s) 204, communication interface device(s), alternatively referred as input/output (I/O) interface(s) 206, and one or more data storage devices or a memory 202 operatively coupled to the processor(s) 204. The master device 102 with one or more hardware processors is configured to execute functions of one or more functional blocks of the master device 102. Referring to the components of the master device 102, in an embodiment, the processor(s) 204, can be one or more hardware processors 204. In an embodiment, the one or more hardware processors 204 can be implemented as one or more microprocessors, microcomputers, microcontrollers, digital signal processors, central processing units, state machines, logic circuitries, and/or any devices that manipulate signals based on operational instructions. Among other capabilities, the one or more hardware processors 204 are configured to fetch and execute computer-readable instructions stored in the memory 202. In an embodiment, the system 100 can be implemented in a variety of computing systems including laptop computers, notebooks, hand-held devices such as mobile phones, workstations, mainframe computers, servers, and the like.

The I/O interface(s) 206 can include a variety of software and hardware interfaces, for example, a web interface, a graphical user interface to display the generated target images and the like and can facilitate multiple communications within a wide variety of networks N/W and protocol types, including wired networks, for example, LAN, cable, etc., and short range and long range wireless networks, such as WLAN, cellular, Wi-Fi, Bluetooth and the like. In an embodiment, the I/O interface (s) 206 can include one or more ports for connecting to a number of external devices or to another server such as cloud server 106 or devices. The I/O interface 206 enables short range communication enabling the master device 102 to have a two way connection with the mask for receiving the sensed biologicals parameters and the environmental parameters and sharing the customized health risk score computed based on the parameter values for the ambient environment the wearer of the mask is in. In an embodiment, the master device 102 may utilize the cloud servers such as cloud server 106 for computationally intensive processing to derive health insights from historical data comprising customized risk scores, the meta data of the wearer, the biological parameters, the environmental parameters.

The memory 202 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes.

Further, the memory 202 may include an AI based Real time risk calculation module 210 that is configured to compute the customized risk score for the wearer in the current ambient environment. Further, memory 202 includes a database 208 that stores the received plurality of biological parameters, the plurality of environmental parameters, the computed customized risk scores, the generated alerts and the like. The memory 202 may comprise information pertaining to input(s)/output(s) of each step performed by the processor(s) 204 of the master device 102 and methods of the present disclosure. In a configuration, the database 208 may be external (not shown) to the master device 102 and coupled via the I/O interface 206. Functions of the components of the master device 102 are explained in conjunction with flow diagram of FIG. 5 and system of FIG. 1.

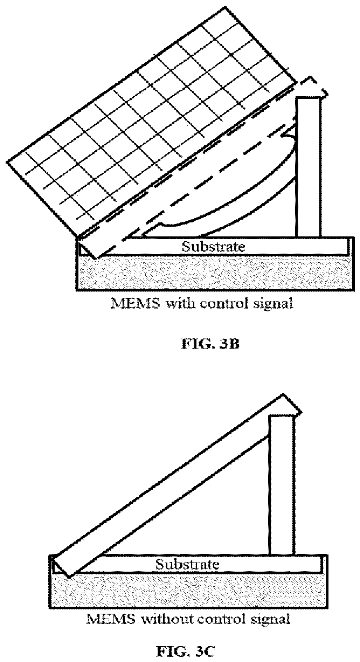

FIG. 3A through 3C (collectively referred as FIG. 3) is an illustrative example of a single layered mesh network used in the mask of the system of FIG. 1, in accordance with some embodiments of the present disclosure.

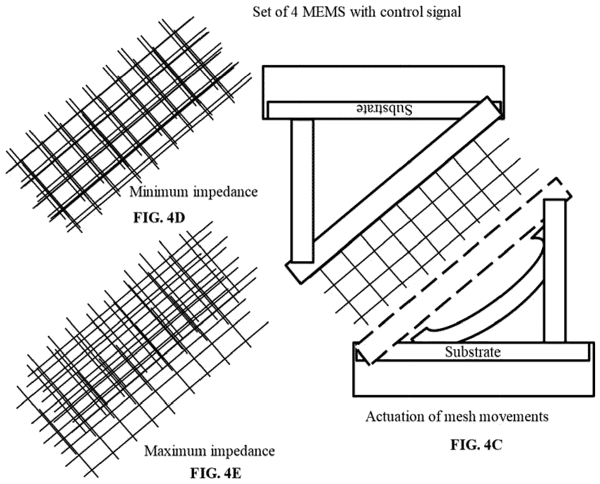

FIG. 4A through 4E (collectively referred as FIG. 4) is an illustrative example of a multi-layered mesh network used in the mask of the system of FIG. 1, in accordance with some embodiments of the present disclosure.

The design and working of single layered and multi-layered mesh is based on same principle. Explained below is the example design of four layered mesh of FIG. 4.

The dimensional aspects of pollutants and mask: Typical sizes of pollen grains is from 15 to 200 micrometers and bacteria are from 0.3 to 2 micrometers, Suspended Particulate matter (SPM) is approx. 2.5 micrometers, whereas viruses are from 0.5 to 300 nanometers. Surgical masks use non-woven material whose average pore size is 0.3 to 10 micrometers where as N95 masks have an average pore size of 0.1 to 0.3 micrometers. Additionally, there is a layer of statically charged material that helps in trapping the pathogens / pollutants.

Consider a grid of filtering material (layer) with average pore size of 1 micrometer. As depicted in FIG. 4A Consider 4 such grids overlaid on each other to form the mesh network 102a. Each layer can be moved precisely by using MEMS (actuators) technology as shown in FIG. 4B. The movement is proportional to the voltage stimulus given to MEMS actuator for that layer. As seen is the FIG. 4C, there are 4 pairs of MEMS actuators coupled with 4 layers of filter material.

Assume that activation of 1st MEMS displace the filter1 by 20 microns overlapping other 3, 2nd MEMS actuation displaces filter 2 by 40 microns, 3rd MEMS displaces the filter by 60 microns and 4th MEMs displaces by 80 micrometer. Following table 1 depicts a typical control signal configuration and resultant air impedance of the mesh network 102a.

**TABLE1:**

| Threat | | MEMS1 activation | MEMS2 activation | MEMS 3 activation | MEMS4 activation | Remark |
|---|---|---|---|---|---|---|
| No | | X | X | X | X | Whole 100 micrometer per opening -0 impedance |
| Pollen grain | | Y | X | X | X | 20 % of the average hole size closed |
| Bacterial contamination n/ pathogenic droplets | | y | y | X | X | 40 % of the average hole size closed |
| Viral / SPM contamination detected | | Y | y | y | y | 80 % of the average hole size closed, maximum air impedance |

FIG. 5 is a flow diagram illustrating a method 500 for context aware networked mask with dynamic air impedance for optimum breathability, using the system of FIG. 1, in accordance with some embodiments of the present disclosure.

In an embodiment, the master device 102 comprises one or more data storage devices or the memory 202 operatively coupled to the processor(s) 204 and is configured to store instructions for execution of steps of the method 500 by the processor(s) or one or more hardware processors 204. The steps of the method 500 of the present disclosure will now be explained with reference to the components or blocks of the master device 102 and the system 100 as depicted in FIG. 1 and the steps of flow diagram as depicted in FIG. 5. Although process steps, method steps, techniques or the like may be described in a sequential order, such processes, methods, and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

Referring to the steps of the method 500, at step 502 of the method 500, the one or more hardware processors 204 receive the plurality of environmental parameters and the biological parameters sensed by the plurality of sensors 102d of the mask 102. On receiving the sensed parameters, at step 504 of the method 500, the one or more hardware processors 204 process via the AI based Real time risk calculation module 210 implemented by the one or more hardware processors 204, the plurality of environmental parameters, the biological parameters, ambient environment and context data of a wearer of the mask, meta data of the wearer and user inputs to generate a customized risk score. In one of the embodiments the AI based Real time risk calculation module 210 can be a Deep Neural Network (DNN) pre-trained on large multi-variate time series dataset of biological sensors including CO2 sensors, temperature sensors, SPO2 and moisture sensors. Another DNN can be pre-trained on MEMS, gyroscope, mobility, location etc. time series data. Different such models can be trained as a multi-task learning model that gives all prediction from the same model. During deployment such a DNN or a set of ensemble DNN may be transfer-learned from user context and the particular geographical location, place where the user will be staying for a period. This assumes that during provisioning, the server side has an estimate of pollution, pathogen etc. levels for the location where the mask will be used initially and the combination of mask, phone, wearable etc. that user starts with. If the mask moves to a completely new geography, OR an completely new combination of mask, phone, wearable (user devices) a new updated model needs to be provisioned in the mask. An example of DNN for multivariate time-series can be ResNet. In one of the embodiments for risk calculation predefined thresholds are used and two clusters are defined for location based pathogen level [low, high] and two clusters for the user's mobility towards a given zone as [fast, slow]. For a tuple [pathogen: high, mobility:fast] , the mask immediately starts actuation workflow. For the other case the mask sends alerts to user devices at different time resolutions based on the combined risk level. This can be overridden by a set of predefined rules (e.g. mask user is aware and sets a do not disturb to the mask software through phone UI). The actual provisioning of the DNN involves a capability (processing, memory, battery) based partitioning of the DNN model, where each part of the model is be mapped to the device hierarchy (eg. mask → smartphone → edge server). The server side database is asynchronously updated by the pollution/pathogen levels sent by the mask users in a geographical location, and the server averages the levels obtained from the mask-users.

At step 506 of the method 500, the one or more hardware processors 204 communicates the customized risk score to the actuator unit 102c2 of the mask 102. According to the invention, the actuator unit 102c2 processes the customized risk score to generate the actuation signal that varies the stretch force applied by the actuator 102b on each of one or more layers of the mesh network 102a in accordance with the customized risk score to vary effective pore size (pore diameter) of the mesh network 102a, which in turn varies the resultant air impedance of the mesh network 102a. Thus, enables adjusting breathability for the wearer.

At step 508 of the method 500, the one or more hardware processors 204 generate alerts for the wearer in accordance with the customized risk score and a set of predefined rules. The example rules are stated above at step 504 in context of AI based risk module generation/ training.

At step 510 of the method 500, the one or more hardware processors 204 communicate with the cloud server 106 for computationally intensive processing to derive health insights from historical data comprising customized risk scores, the meta data of the wearer, the biological parameters, the environmental parameters. Thus, large volume of data collected over time from the smart mask and the computed risk scores, trained AL modules and analytical tools in the cloud server can health insights and predictions for the wearer , which can be provided on the master device 104 as notifications and reports.

Masks are essential survival tool in case of immune-compromised patients or in case of pandemics. One of the challenges of using mask for extended time is breathability. Less breathability leads to uncomforted and possible hypoxia. Smart masks available in the art hardly focus smartness with breathability context. The embodiments disclosed herein provide mechanism to have a dynamic impedance for breathability of mask depending on the context. Context is determined by using AI techniques with input variables such as sensors data, wearers metadata, location and so on. The system disclosed leverages capability of MEMS as actuating mechanism to manipulate the layers with uniform micro sized pores to achieve variability in breathability impedance.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g. hardware means like e.g. an application-specific integrated circuit (ASIC), a field-programmable gate array (FPGA), or a combination of hardware and software means, e.g. an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g. using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A system (100) for context aware networked smart mask with dynamic air impedance for optimum breathability, the system comprising:
a mask (102) comprising a base material (102f) attached with holding strings (102e) at two ends, wherein the base material (102f) holds:
a mesh network (102a) having one or more layers with a pore diameter associated with each of the one or more layers, wherein each of the one or more layers is held by an actuator (102b) applying a stretch force on each of the one or more layers;
a plurality of sensors (102d), attached to the base material (102f), and comprising a plurality of air quality sensors for sensing a plurality of environmental parameters and a plurality of biological sensors for sensing a plurality of biological parameters of a wearer of the mask;
a communication interface (102c1) attached to the base material (102f) and configured to a) transmit the sensed plurality of environmental parameters and the plurality of biological parameters to a master device (104) and b) receive a customized risk score from the master device; and
an actuator unit (102c2) configured to process the customized risk score received from the communication module (102c1) to generate an actuation signal that varies the stretch force applied by the actuator (102b) on each of the one or more layers in accordance to the customized risk score to vary resultant air impedance of the mesh network by varying an effective pore diameter to adjust breathability for the wearer;
the master device (104), comprising:
a memory (202) storing instructions;
one or more Input/Output (I/O) interfaces (206); and
one or more hardware processors (204) coupled to the memory (202) via the one or more I/O interfaces (206), wherein the one or more hardware processors (204) are configured by the instructions to:
process, via an AI based Real time risk calculation module (210) in the memory (202) implemented by the one or more hardware processors (204), a) the plurality of environmental parameters, the plurality of biological parameters, ambient environment and context data of the wearer, meta data of the wearer and user inputs to generate the customized risk score, and b) generate alerts for the wearer in accordance to the customized risk score and a set of predefined rules.

2. The system as claimed in claim 1, wherein the master device (104) is configured to communicate with a cloud server 106 for deriving health insights from historical data comprising customized risk scores, the meta data of the wearer, the plurality of biological parameters, the plurality of environmental parameters.

3. The system as claimed in claim 1, wherein the air quality sensors comprise pathogen sensors, pollen grain sensors, Volatile Organic Compounds (VOC) sensors, bacteria sensors, ear lobe based PPG sensors and mold sensors.

4. The system as claimed in claim 1, wherein the biological sensors comprise CO2 sensors, temperature sensors, SPO2 and moisture sensors.

5. The system as claimed in claim 1, wherein the actuator is a precision actuator comprising at least one of a Micro ElectroMechanical System (MEMS), piezoelectric crystals or bimetallic strips.

6. The system as claimed in claim 1, wherein the mesh network comprises one of
a mesh of closely knit polymer with elastic properties, wherein the effective pore diameter of the closely knit polymer increases with the increasing stretch force applied by the actuator unit (102c2) allowing more air to enter the mask for increased breathability; and
a multi-layered mesh with pore sizes larger than the closely knit polymer, wherein layers of the mesh are overlaid on each other, and wherein the actuator unit is configured to realign the pores of each layer in accordance with the actuator control signal to vary the resultant pore size of the multi-layered mesh changing the air impedance and modulating the breathability.

7. A processor implemented method for context aware networked smart mask with dynamic air impedance for optimum breathability, the method comprising:
receiving (502), by one or more hardware processors of a master device, a plurality of environmental parameters and a plurality of biological parameters sensed by a plurality of sensors of a mask;
processing (504), via an AI based Real time risk calculation module implemented by the one or more hardware processors, the plurality of environmental parameters, the plurality of biological parameters, ambient environment and context data of a wearer of the mask, meta data of the wearer and user inputs to generate a customized risk score;
communicating (506), via the one or more hardware processors, the customized risk score to an actuator unit of the mask, wherein actuator unit processes the customized risk score to generate an actuation signal that varies a stretch force applied by an actuator on each of one or more layers of a mesh network of the mask in accordance with the customized risk score to vary effective pore diameter of the mesh network, which in turn varies resultant air impedance to adjust breathability for the wearer; and
generate (508), via the one or more hardware processors, alerts for the wearer in accordance with the customized risk score and a set of predefined rules.

8. The method as claimed in claim 7, wherein the method further comprising communicating (510) with a cloud server for deriving health insights from historical data comprising customized risk scores, the meta data of the wearer, the plurality of biological parameters, the plurality of environmental parameters.

9. One or more non-transitory machine-readable information storage mediums comprising one or more instructions, which when executed by one or more hardware processors causes a method context aware networked smart mask with dynamic air impedance for optimum breathability, the method comprising:
receiving, by one or more hardware processors of a master device, a plurality of environmental parameters and a plurality of biological parameters sensed by a plurality of sensors of a mask;
processing, via an AI based Real time risk calculation module implemented by the one or more hardware processors, the plurality of environmental parameters, the plurality of biological parameters, ambient environment and context data of a wearer of the mask, meta data of the wearer and user inputs to generate a customized risk score;
communicating, via the one or more hardware processors, the customized risk score to an actuator unit of the mask, wherein actuator unit processes the customized risk score to generate an actuation signal that varies a stretch force applied by an actuator on each of one or more layers of a mesh network of the mask in accordance with the customized risk score to vary effective pore diameter of the mesh network, which in turn varies resultant air impedance to adjust breathability for the wearer; and
generate, via the one or more hardware processors, alerts for the wearer in accordance with the customized risk score and a set of predefined rules.

10. The one or more non-transitory machine-readable information storage mediums of claim 9, further comprising communicating with a cloud server 106 for deriving health insights from historical data comprising customized risk scores, the meta data of the wearer, the plurality of biological parameters, the plurality of environmental parameters.

## Patentansprüche

1. System (100) für kontextbewusste vernetzte intelligente Maske mit dynamischer Luftimpedanz für optimale Atmungsaktivität, wobei das System umfasst:
eine Maske (102), die ein Basismaterial (102f) umfasst, das mit Haltebändern (102e) an zwei Enden befestigt ist, wobei das Basismaterial (102f) aufweist:
ein Maschennetz (102a) mit einer oder mehreren Schichten mit einem Porendurchmesser, der mit jeder der einen oder mehreren Schichten assoziiert ist, wobei jede der einen oder mehreren Schichten durch einen Aktor (102b) gehalten wird, der eine Streckkraft auf jede der einen oder mehreren Schichten anwendet;
eine Vielzahl von Sensoren (102d), die an dem Basismaterial (102f) befestigt sind, und umfassend eine Vielzahl von Luftqualitätssensoren zum Abfühlen einer Vielzahl von Umweltparametern und eine Vielzahl von biologischen Sensoren zum Abfühlen einer Vielzahl von biologischen Parametern eines Trägers der Maske;
eine Kommunikationsschnittstelle (102c1), die an dem Basismaterial (102f) befestigt ist und ausgestaltet ist, um a) die abgefühlte Vielzahl von Umweltparametern und die Vielzahl von biologischen Parametern an eine Mastervorrichtung (104) zu übertragen und b) einen benutzerspezifischen Risiko-Score von der Mastervorrichtung zu empfangen; und
eine Aktoreinheit (102c2), die ausgestaltet ist, um den benutzerspezifischen Risiko-Score zu verarbeiten, der von dem Kommunikationsmodul (102c1) empfangen wurde, um ein Betätigungssignal zu generieren, das die durch den Aktor (102b) auf jede der einen oder mehreren Schichten angewendete Streckkraft gemäß dem benutzerspezifischen Risiko-Score variiert, um die resultierende Luftimpedanz des Maschennetzes zu variieren, indem ein effektiver Porendurchmesser variiert wird, um die Atmungsaktivität für den Träger anzupassen;
wobei die Mastervorrichtung (104) umfasst:
einen Speicher (202), der Anweisungen speichert;
eine oder mehrere Eingabe/Ausgabe (I/0)-Schnittstellen (206); und
einen oder mehrere Hardwareprozessoren (204), die über die eine oder mehreren I/O-Schnittstellen (206) an den Speicher (202) gekoppelt sind, wobei der eine oder die mehreren Hardwareprozessoren (204) durch die Anweisungen ausgestaltet ist/sind zum:
Verarbeiten über ein Echtzeit-Risikoberechnungsmodul (210) auf AI-Basis in dem Speicher (202), das durch den einen oder die mehreren Hardwareprozessoren (204) implementiert wird, von a) der Vielzahl von Umweltparametern, der Vielzahl von biologischen Parametern, Umweltumgebungs- und Kontextdaten des Trägers, Metadaten des Trägers und Benutzereingaben, um den benutzerspezifischen Risiko-Score zu generieren, und b) Alarmmeldungen für den Träger gemäß dem benutzerspezifischen Risiko-Score und einem Satz vordefinierter Regeln zu generieren.

2. System nach Anspruch 1, wobei die Mastervorrichtung (104) ausgestaltet ist, um mit einem Cloud-Server (106) zu kommunizieren, um gesundheitliche Erkenntnisse aus historischen Daten abzuleiten, die benutzerspezifische Risiko-Scores, die Metadaten des Trägers, die Vielzahl der biologischen Parameter, die Vielzahl der Umweltparameter umfassen.

3. System nach Anspruch 1, wobei die Luftqualitätssensoren Pathogensensoren, Pollenkornsensoren, Sensoren auf flüchtige organische Verbindungen (VOC), Bakteriensensoren, PPG-Sensoren auf Ohrläppchenbasis und Schimmelsensoren umfassen.

4. System nach Anspruch 1, wobei die biologischen Sensoren CO₂-Sensoren, Temperatursensoren, SPO₂- und Feuchtigkeitssensoren umfassen.

5. System nach Anspruch 1, wobei der Aktor ein Präzisionsaktor ist, der mindestens eines von einem Mikro-ElektroMechanischen System (MEMS), piezoelektrischen Kristallen oder Bimetallstreifen umfasst.

6. System nach Anspruch 1, wobei das Maschennetz eines der folgenden umfasst:
ein Maschenmaterial aus dicht gestricktem Polymer mit elastischen Eigenschaften, wobei der effektive Porendurchmesser des dicht gestrickten Polymers mit der zunehmenden Streckkraft zunimmt, die durch die Aktoreinheit (102c2) angewendet wird, wodurch zugelassen wird, dass für erhöhte Atmungsaktivität mehr Luft in die Maske eintritt; und
ein mehrschichtiges Maschenmaterial mit Porengrö-ßen größer als denjenigen des dicht gestrickten Polymers, wobei Schichten des Maschenmaterials übereinander gelegt sind, und wobei die Aktoreinheit ausgestaltet ist, um die Poren jeder Schicht gemäß dem Aktorsteuersignal erneut auszurichten, um die resultierende Porengröße des mehrschichtigen Maschenmaterials zu variieren, wodurch die Luftimpedanz geändert und die Atmungsaktivität moduliert wird.

7. Prozessorimplementiertes Verfahren für kontextbewusste vernetzte intelligente Maske mit dynamischer Luftimpedanz für optimale Atmungsaktivität, wobei das Verfahren umfasst:
Empfangen (502) einer Vielzahl von Umweltparametern und einer Vielzahl von biologischen Parametern, die durch eine Vielzahl von Sensoren einer Maske abgefühlt werden, durch einen oder mehrere Hardwareprozessoren einer Mastervorrichtung;
Verarbeiten (504) der Vielzahl von Umweltparametern, der Vielzahl von biologischen Parametern, Umweltumgebungs- und Kontextdaten eines Trägers der Maske, Metadaten des Trägers und Benutzereingaben über ein Echtzeit-Risikoberechnungsmodul auf AI-Basis, das durch den einen oder die mehreren Hardwareprozessoren implementiert wird, um einen benutzerspezifischen Risiko-Score zu generieren;
Kommunizieren (506) des benutzerspezifischen Risiko-Scores über den einen oder die mehreren Hardwareprozessoren an eine Aktoreinheit der Maske, wobei die Aktoreinheit den benutzerspezifischen Risiko-Score verarbeitet, um ein Betätigungssignal zu generieren, das eine Streckkraft variiert, die durch einen Aktor auf jede der einen oder mehreren Schichten eines Maschennetzes der Maske gemäß dem benutzerspezifischen Risiko-Score angewendet wird, um den effektiven Porendurchmesser des Maschennetzes zu variieren, das wiederum die resultierende Luftimpedanz variiert, um die Atmungsaktivität für den Träger anzupassen; und
Generieren (508) von Alarmmeldungen für den Träger gemäß dem benutzerspezifischen Risiko-Score und einem Satz vordefinierter Regeln über den einen oder die mehreren Hardwareprozessoren.

8. Verfahren nach Anspruch 7, wobei das Verfahren des Weiteren Kommunizieren (510) mit einem Cloud-Server umfasst, um gesundheitliche Erkenntnisse aus historischen Daten abzuleiten, die benutzerspezifische Risiko-Scores, die Metadaten des Trägers, die Vielzahl der biologischen Parameter, die Vielzahl der Umweltparameter umfassen.

9. Ein oder mehrere nicht-flüchtige maschinenlesbare Informationsspeicherungsmedien, umfassend eine oder mehrere Anweisungen, die bei Ausführung durch einen oder mehrere Hardwareprozessoren ein Verfahren mit kontextbewusster vernetzter intelligenter Maske mit dynamischer Luftimpedanz für optimale Atmungsaktivität bewirkt/bewirken, wobei das Verfahren umfasst:
Empfangen einer Vielzahl von Umweltparametern und einer Vielzahl von biologischen Parametern, die durch eine Vielzahl von Sensoren einer Maske abgefühlt werden, durch einen oder mehrere Hardwareprozessoren einer Mastervorrichtung;
Verarbeiten der Vielzahl von Umweltparametern, der Vielzahl von biologischen Parametern, Umweltumgebungs- und Kontextdaten eines Trägers der Maske, Metadaten des Trägers und Benutzereingaben über ein Echtzeit-Risikoberechnungsmodul auf AI-Basis, das durch den einen oder die mehreren Hardwareprozessoren implementiert wird, um einen benutzerspezifischen Risiko-Score zu generieren;
Kommunizieren des benutzerspezifischen Risiko-Scores über den einen oder die mehreren Hardwareprozessoren an eine Aktoreinheit der Maske, wobei die Aktoreinheit den benutzerspezifischen Risiko-Score verarbeitet, um ein Betätigungssignal zu generieren, das eine Streckkraft variiert, die durch einen Aktor auf jede der einen oder mehreren Schichten eines Maschennetzes der Maske gemäß dem benutzerspezifischen Risiko-Score angewendet wird,
um den effektiven Porendurchmesser des Maschennetzes zu variieren, das wiederum die resultierende Luftimpedanz variiert, um die Atmungsaktivität für den Träger anzupassen; und
Generieren von Alarmmeldungen für den Träger gemäß dem benutzerspezifischen Risiko-Score und einem Satz vordefinierter Regeln über den einen oder die mehreren Hardwareprozessoren.

10. Ein oder mehrere nicht-flüchtige maschinenlesbare Informationsspeichermedien nach Anspruch 9, des Weiteren umfassend Kommunizieren mit einem Cloud-Server (106), um gesundheitliche Erkenntnisse aus historischen Daten abzuleiten, die benutzerspezifische Risiko-Scores, die Metadaten des Trägers, die Vielzahl der biologischen Parameter, die Vielzahl der Umweltparameter umfassen.

## Revendications

1. Système (100) pour masque intelligent en réseau sensible au contexte à impédance d'air dynamique pour respirabilité optimale, le système comprenant :
un masque (102) comprenant un matériau de base (102f) fixé avec des cordelettes de maintien (102e) à deux extrémités, le matériau de base (102f) maintenant :
un réseau maillé (102a) ayant une ou plusieurs couches avec un diamètre de pore associé à chacune de la ou des couches, chacune de la ou des couches étant maintenue par un actionneur (102b) appliquant une force d'étirement sur chacune de la ou des couches ;
une pluralité de capteurs (102d), fixés au matériau de base (102f), et comprenant une pluralité de capteurs de qualité de l'air pour détecter une pluralité de paramètres environnementaux, et une pluralité de capteurs biologiques pour détecter une pluralité de paramètres biologiques d'un porteur du masque ;
une interface de communication (102c1) fixée au matériau de base (102f) et configurée pour a) transmettre la pluralité de paramètres environnementaux et la pluralité de paramètres biologiques détectés à un dispositif maître (104) et b) recevoir un score de risque personnalisé du dispositif maître ; et
une unité d'actionneur (102c2) configurée pour traiter le score de risque personnalisé reçu du module de communication (102c1) pour générer un signal d'actionnement qui fait varier la force d'étirement appliquée par l'actionneur (102b) sur chacune de la ou des couches en fonction du score de risque personnalisé pour faire varier l'impédance d'air résultante du réseau maillé en faisant varier un diamètre de pore effectif pour ajuster la respirabilité pour le porteur ;
le dispositif maître (104), comprenant :
une mémoire (202) stockant des instructions ;
une ou plusieurs interfaces d'entrée/sortie (E/S) (206) ; et
un ou plusieurs processeurs matériels (204) couplés à la mémoire (202) par l'intermédiaire de la ou des interfaces E/S (206), le ou les processeurs matériels (204) étant configurés par les instructions pour :
traiter, par l'intermédiaire d'un module de calcul de risque en temps réel basé sur l'IA (210) dans la mémoire (202) mis en oeuvre par le ou les processeurs matériels (204), a) la pluralité de paramètres environnementaux, la pluralité de paramètres biologiques, l'environnement ambiant et les données de contexte du porteur, les métadonnées du porteur et les entrées de l'utilisateur pour générer le score de risque personnalisé, et b) générer des alertes pour le porteur conformément au score de risque personnalisé et à un ensemble de règles prédéfinies.

2. Système selon la revendication 1, le dispositif maître (104) étant configuré pour communiquer avec un serveur en nuage (106) pour dériver des informations sur la santé à partir de données historiques comprenant des scores de risque personnalisés, les métadonnées du porteur, la pluralité de paramètres biologiques, la pluralité de paramètres environnementaux.

3. Système selon la revendication 1, les capteurs de qualité de l'air comprenant des capteurs de pathogènes, des capteurs de grains de pollen, des capteurs de composés organiques volatils (COV), des capteurs de bactéries, des capteurs PPG basés sur le lobe de l'oreille et des capteurs de moisissures.

4. Système selon la revendication 1, les capteurs biologiques comprenant des capteurs de CO2, des capteurs de température, des capteurs de SPO2 et des capteurs d'humidité.

5. Système selon la revendication 1, l'actionneur étant un actionneur de précision comprenant au moins un système micro-électromécanique (MEMS), des cristaux piézoélectriques ou des bandes bimétalliques.

6. Système selon la revendication 1, le réseau maillé comprenant l'un des éléments suivants :
un maillage de polymère à mailles serrées ayant des propriétés élastiques, le diamètre effectif des pores du polymère à mailles serrées augmentant avec l'augmentation de la force d'étirement appliquée par l'unité d'actionnement (102c2) permettant à davantage d'air de pénétrer dans le masque pour une respirabilité accrue ; et
un maillage multicouche avec des tailles de pores plus grandes que le polymère à mailles serrées, des couches du maillage étant superposées les unes sur les autres, et l'unité d'actionnement étant configurée pour réaligner les pores de chaque couche en fonction du signal de commande de l'actionneur pour faire varier la taille de pore résultante du maillage multicouche, changeant l'impédance de l'air et modulant la respirabilité.

7. Procédé mis en œuvre par un processeur pour masque intelligent en réseau sensible au contexte à impédance d'air dynamique pour respirabilité optimale, le procédé comprenant :
la réception (502), par un ou plusieurs processeurs matériels d'un dispositif maître, d'une pluralité de paramètres environnementaux et d'une pluralité de paramètres biologiques détectés par une pluralité de capteurs d'un masque ;
le traitement (504), par l'intermédiaire d'un module de calcul de risque en temps réel basé sur l'IA et mis en oeuvre par le ou les processeurs matériels, de la pluralité de paramètres environnementaux, de la pluralité de paramètres biologiques, de l'environnement ambiant et des données de contexte d'un porteur du masque, des métadonnées du porteur et des entrées de l'utilisateur pour générer un score de risque personnalisé ;
la communication (506), par l'intermédiaire du ou des processeurs matériels, du score de risque personnalisé à une unité d'actionnement du masque, l'unité d'actionnement traitant le score de risque personnalisé pour générer un signal d'actionnement qui fait varier une force d'étirement appliquée par un actionneur sur chacune d'une ou plusieurs couches d'un réseau maillé du masque conformément au score de risque personnalisé pour faire varier le diamètre effectif des pores du réseau maillé, qui à son tour fait varier l'impédance d'air résultante pour ajuster la respirabilité pour le porteur ; et
générer (508), par l'intermédiaire du ou des processeurs matériels, des alertes pour le porteur en fonction du score de risque personnalisé et d'un ensemble de règles prédéfinies.

8. Procédé selon la revendication 7, le procédé comprenant en outre la communication (510) avec un serveur en nuage pour dériver des informations sur la santé à partir de données historiques comprenant des scores de risque personnalisés, les métadonnées du porteur, la pluralité de paramètres biologiques, la pluralité de paramètres environnementaux.

9. Support(s) de stockage d'informations non transitoires lisibles par machine comprenant une ou plusieurs instructions qui, lorsqu'elles sont exécutées par un ou plusieurs processeurs matériels, amènent un procédé de masque intelligent en réseau sensible au contexte à impédance d'air dynamique pour respirabilité optimale, le procédé comprenant :
la réception, par un ou plusieurs processeurs matériels d'un dispositif maître, d'une pluralité de paramètres environnementaux et d'une pluralité de paramètres biologiques détectés par une pluralité de capteurs d'un masque ;
le traitement, par l'intermédiaire d'un module de calcul de risque en temps réel basé sur l'IA et mis en oeuvre par un ou plusieurs processeurs matériels, de la pluralité de paramètres environnementaux, de la pluralité de paramètres biologiques, de l'environnement ambiant et des données contextuelles d'un porteur du masque, des métadonnées du porteur et des entrées utilisateur pour générer un score de risque personnalisé ;
la communication, par l'intermédiaire du ou des processeurs matériels, du score de risque personnalisé à une unité d'actionnement du masque, l'unité d'actionnement traitant le score de risque personnalisé pour générer un signal d'actionnement qui fait varier une force d'étirement appliquée par un actionneur sur chacune d'une ou plusieurs couches d'un réseau maillé du masque conformément au score de risque personnalisé pour faire varier le diamètre effectif des pores du réseau maillé, qui à son tour fait varier l'impédance d'air résultante pour ajuster la respirabilité pour le porteur ; et
générer, par l'intermédiaire du ou des processeurs matériels, des alertes pour le porteur en fonction du score de risque personnalisé et d'un ensemble de règles prédéfinies.

10. Support(s) de stockage d'informations lisibles par machine non transitoires selon la revendication 9, comprenant en outre la communication avec un serveur en nuage (106) pour dériver des informations sur la santé à partir de données historiques comprenant des scores de risque personnalisés, les métadonnées du porteur, la pluralité de paramètres biologiques, la pluralité de paramètres environnementaux.
